# EUROPEAN PATENT SPECIFICATION

(11) **EP 2 143 435 B1**
(45) Date of publication and mention of the grant of the patent: **11.04.2012**
(21) Application number: 08753884.9
(22) Date of filing: 26.03.2008
(51) Int. Cl.: A61K 36/15, A61K 31/085, A61K 31/352, B01D 11/02

(54) **METHOD FOR EXTRACTING SECOISOLARICIRESINOL AND DIHYDROQUERCETIN FROM WOOD**
VERFAHREN ZUR EXTRAKTION VON SECOISOLARICIRESINOL UND DIHYDROQUERCETIN AUS HOLZ
PROCÉDÉ D'EXTRACTION DE SECOISOLARICIRESINOL ET DE DIHYDROQUERCÉTINE À PARTIR DU BOIS

(30) Priority: 29.03.2007 RU 2007111534
(43) Date of publication of application: 13.01.2010
(73) Proprietor: Nifantiev, Nikolay Eduardovich, Moscow 117593 (RU)
(72) Inventor: YASHUNSKY, Dmitri Vladimirovich, Moscow, 119270 (RU); MENSHOV, Vladimir Mikhailovich, Moscow, 117321 (RU); TSVETKOV, Yury Evgenievich, Moscow, 119421 (RU); TSVETKOV, Dmitri Evgenievich, Moscow, 117312 (RU)
(74) Representative: Corradini, Corrado
(86) International application number: PCT/RU2008/000176
(87) International publication number: WO 2008/121021

(56) References cited:
- WO-A1-02/098830
- WO-A1-03/020254
- WO-A1-2005/047423
- RU-C1- 2 088 256
- RU-C1- 2 154 967
- RU-C2- 2 288 582
- US-A1- 2004 199 032
- 'Indol-3-Karbinol Perechen dannkh', [Online] 2004, pages 1 - 2, XP008120966 Retrieved from the Internet: <URL:http://www.vitashop.ru/c7126/pid6902>

## Description

### Background Art

The present invention concerns the area of highly technological forest chemistry and wood refinery.

The invention proposes the method for the isolation from wood of valuable physiologically active compounds, including extracts enriched in lignans and flavonoids (lignan-flavonoid complexes) and separated lignans and flavonoids designed for the subsequent use in the production of biologically active additives and chemical pharmaceutical products.

### Background of the invention

Lignans and flavonoids are classified as phenol compounds composing various parts of plants, for instance, softwood. Chemical extracts containing lignan and flavonoid derivatives and products produced on the basis of these extracts find wide use in medicine and cosmetology, the food industry, as agrochemical preparations in crop production, and in other fields.

One of interesting compounds classified as lignan is secoisolariciresinol (SECO). Dihydroquercetin (DHQ) is produced in the method of this invention as a representative of the class of flavonoids. Dihydroquercetin is often designated in the literature by its synonym: taxifolin.

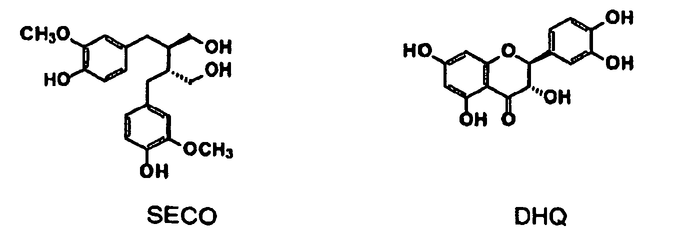

DHQ was studied rather widely in research groups to reveal areas of its practical use. In Russia, DHQ is most widely used in the composition of the whole series of biologically active preparations (for example, the preparations Kapilar, Flukol, and others) and some food products, because of high antioxidant, hepatoprotective, immunostimulating, antimicrobial, and other practically useful properties of this compound. For instance, a regulator of growth and development of crops was created on the basis of DHQ isolated from sawdust of Siberian and Dahurian larches. This preparation has the commercial name Larixin (Russian Patent No. 2229213; Russian Patent No. 2256328).

SECO also represents a very promising preparation for practical use, especially for the production of drugs, biologically active compounds, and functional food products due to the antioxidant, antiphlogistic, phytoextragenic, antimicrobial, and other useful properties of this compound. For example, a pharmaceutical composition (International Patent Publication No. WO03/020254) based on SECO (isolated from the *Stereospermum personatum* plant) is known, as well as the functional food products (International Patent Publication No. WO02/080702), hepatoprotective and anticancer preparations (U.S. Patent Publication No. 2006/0035964), and other preparations including SECO.

It is known that knot zones and branches of conifers contain considerable amounts of phenol compounds (B. Holmbom, C. Eckerman, P. Eklund, J. Hemming, L. Nisula, M. Reunanen, R. Sjöholm, A. Sundberg, K. Sundberg, S. Willför, "Knots in trees - A new rich source of lignans," Phytochemistry Reviews 2: 331-340, 2003). However, the authors of the present invention have experimentally found that only the so-called "white" knots, i.e., knots bearing living branches, are preferential raw materials for the isolation of the compounds of interest in industry (see below, Example 1).

The isolation of phenol compounds from knot zones of wood is known (see U.S. Patent Publication No. 2004/0199032). This publication summarizes the method for the isolation of phenol compounds from wood containing knot zones. In this method, the crushed wood of the knot zone is extracted with a polar solvent followed by the isolation of substances from the extract.

The following technical restrictions can be assigned to disadvantages of the said method. The proposed conditions were developed for the isolation of one target product, a representative of the class of lignans 7-hydroxymatairesinol (HMR). However, the conifer wood usually contains more than one component assigned to phenol compounds, and these components are attributed not only to the lignan class. In addition to them, the wood contains representatives of the flavonoid class, as well as lignan derivatives, which are practically valuable. Economical expedience of processing of this type of wood raw materials requires the development of methods for the simultaneous extraction of compounds belonging to different classes, for instance, lignans and flavonoids, for their further use. However, it turned out that the known method (U.S. Patent Publication No. 2004/0199032) is poorly efficient for the simultaneous isolation of lignans in a mixture with flavonolds, whose solubility profile in organic solvents differs from that of lignans. Another disadvantage of the known method is the necessity of preparing the wood for extraction, including the stages of washing with alkane to remove volatile components followed by drying, being also designed for the removal of water, which is present in the wood and whose presence decreases, most likely, the extracting ability of the solvent. The polar solvent used in the said method was determined generally by indicating the dielectric constant (which should be higher than 3 at 25 °C). Such technologically important solvents as diethyl ether, chloroform, and methylene chloride correspond to these dielectric constant values (their dielectric constants are 4.34, 4.70, and 8.9, respectively). In these solvents DHQ is very poorly soluble, which does not allow one to use these solvents for DHQ extraction.

It was also mentioned in the known method (US 2004/0199032 of October 7, 2004) that an acetone/water mixture can be used as a polar solvent. However, the chosen ratio (95:5 vol.%) of the mixture components does not give DHQ of interest in a sufficient yield (see below Example 2).

The task of the present invention is the development of a method for the isolation of phenol compounds from wood without the indicated disadvantages. The use of the provided method provides broad prospects for the production of a wide scope of biologically active additives, chemical pharmaceutical preparations, and other products.

The stated problem is solved by the instant invention. In the method of the invention, the crushed wood of the knot zone is extracted with a non-splitting mixture of an organic solvent with water, having a content of the organic solvent of 50-75%, to obtain an extract containing SECO and DHQ, and the extract is treated to remove the solvent and obtain the final mixture containing SECO and DHQ. Washing of the crushed wood of the knot zone with alkane, as in the known method (US 2004/0199032 of October 7, 2004), or with a CO₂ flow (including under the conditions of supercritical fluid extraction) makes it possible to extract only the simplest low-molecular-weight, mostly low-polarity organic compounds from the wood mass, which exerts no effect on the efficiency of extraction of a mixture of SECO and DHQ under the experimental conditions of the said method.

In another variant of the invention, the obtained mixture containing SECO and DHQ is additionally subjected to selective extraction and crystallization with isolation of the purified target compounds.

In another variant of the invention, the extract containing secoisolariciresinol and dihydroquercetin is additionally treated to remove nonpolar components, the supernatant liquid is removed, and the resulting residue is chromatographed on a silica gel layer followed by the removal of the eluent to obtain a dry mixture of secoisolariciresinol and dihydroquercetin.

In the methods of this invention, the conifer wood, preferably larch (*Larix*), can be used as the wood; particularly, Siberian (*Larix sibirica*) and Dahurian larch (*Larix dahurica*), as well as fir (*Abies*); particularly, Siberian fir (*Abies sibirica*), and "white knots" are used as the knot zone of the wood.

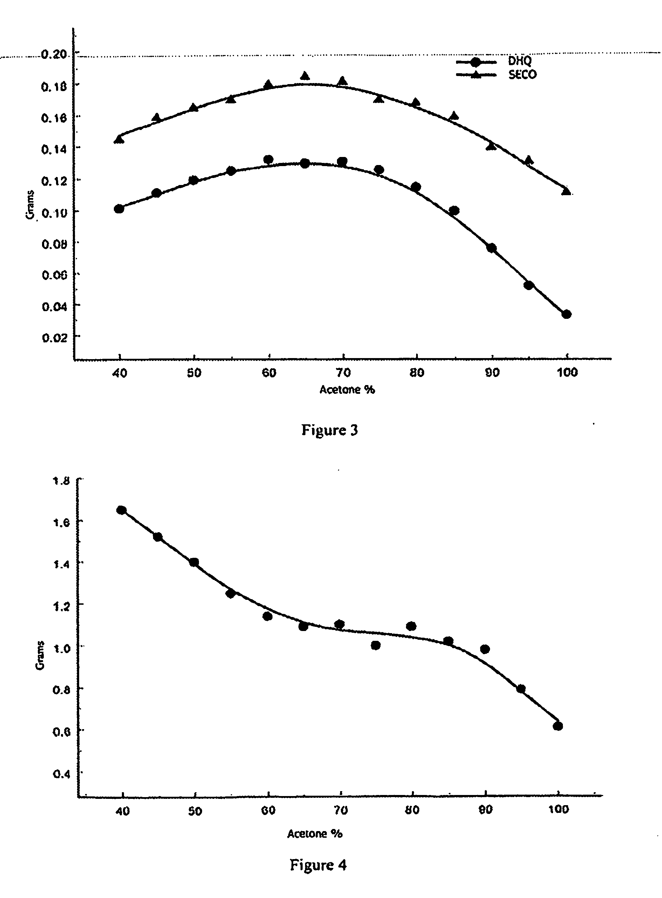

As follows from the data presented in Examples 2 and 3, the highest efficiency of SECO and DHQ extraction is achieved when mixtures of an organic solvent and water are used, whereas the efficiency in a neat organic solvent is lower. The solubility is also lower in neat water (omitted in Figs. 3-8) in which the target compounds are restrictedly soluble (dissolution in water needs heating). Therefore, the most important property of the used organic solvent in this invention is its ability to mix with water in the range of the indicated ratios to give a non-splitting mixture with the required dissolving activity. The analysis of solvents in this invention shows that their polarity is characterized by the dielectric constant values from 10 to 40 at 25-30 °C. Acetone can be used as an organic solvent, and its content in a mixture with water ranges from 50 to 75%, preferably from 60 to 70%.

In another variant of the invention, 2-propanol can be used as an organic solvent, and its content in a mixture with water ranges from 50 to 75%, preferably from 60 to 70%.

In other variants, other solvents, for example, ethanol, satisfying the above characteristics, can be used as an organic solvent, and the content of the solvent in a mixture with water ranges from 50 to 75%, preferably from 60 to 70%.

The key advantage of the present invention is a possibility to provide the efficient simultaneous extraction from the wood mass of both compounds related to lignan and flavonoid series, which are interesting for practical use as a mixture or after separation. An advantage over the known method is also simplicity of isolation of the target compounds in the present invention that requires no special drying of the extracted wood (lyophilic drying is used in the known method), crushing of the wood mass, and washing with alkane before extraction with an aqueous organic mixture.

The yields of DHQ under the conditions of the present invention correspond to the best methods of isolation of this compound from natural objects (for instance, according to the methods described in the following sources: E.E. Nifant'ev, M.P. Koroteev, G.Z. Kaziev, A.A. Uminskii, "Method for Isolation of Dihydroquercetin," Russian Patent No. 2180566, 2001; E.E. Nifant'ev, M.P. Koroteev, G.Z. Kaziev, G.A. Volkov, R.Sh. Gataulin, "Method of Complex Processing of Larch Wood," Russian Patent No. 2233858, 2003). The yield of SECO under the conditions of the present invention substantially exceeds that for extraction from other known natural sources, for example, for the extraction from the *Stereospermum personatum* plant the yield is 0.03% (International Patent Publication No. WO 03/020254), for that from *Carissa edulis* the yield is 0.0004% (Phytochemistry 22: 749 (1983)), and for *Juniperus chinensis* the yield is 0.00245% (Phytochemistry 31: 3659 (1992)).

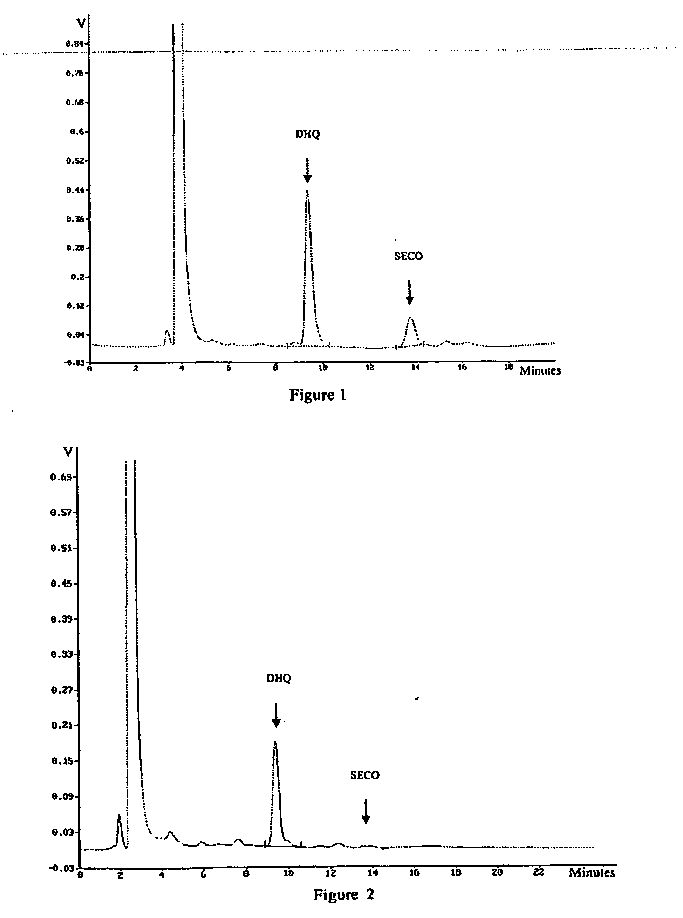

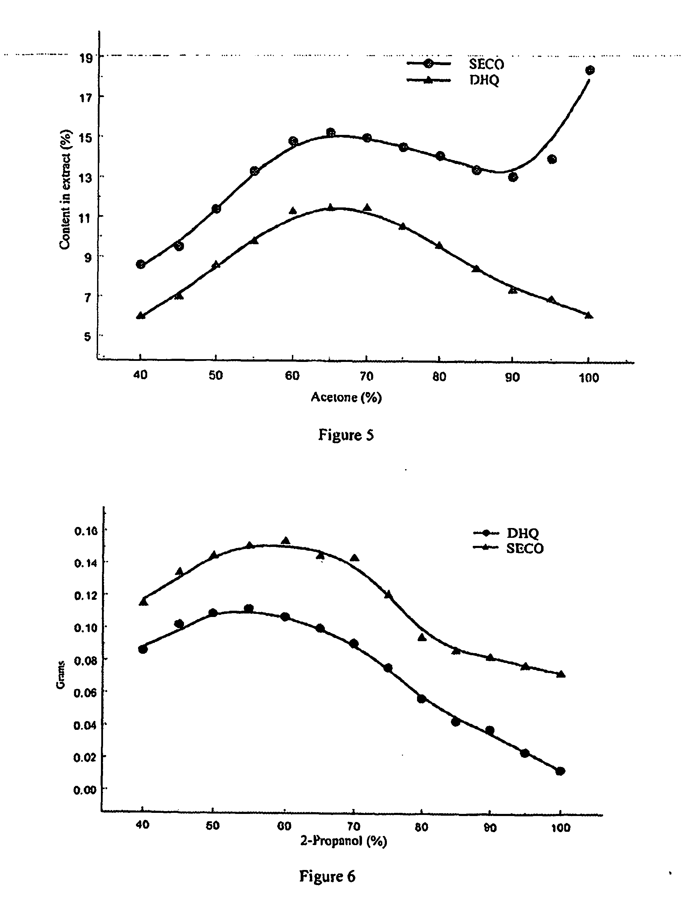

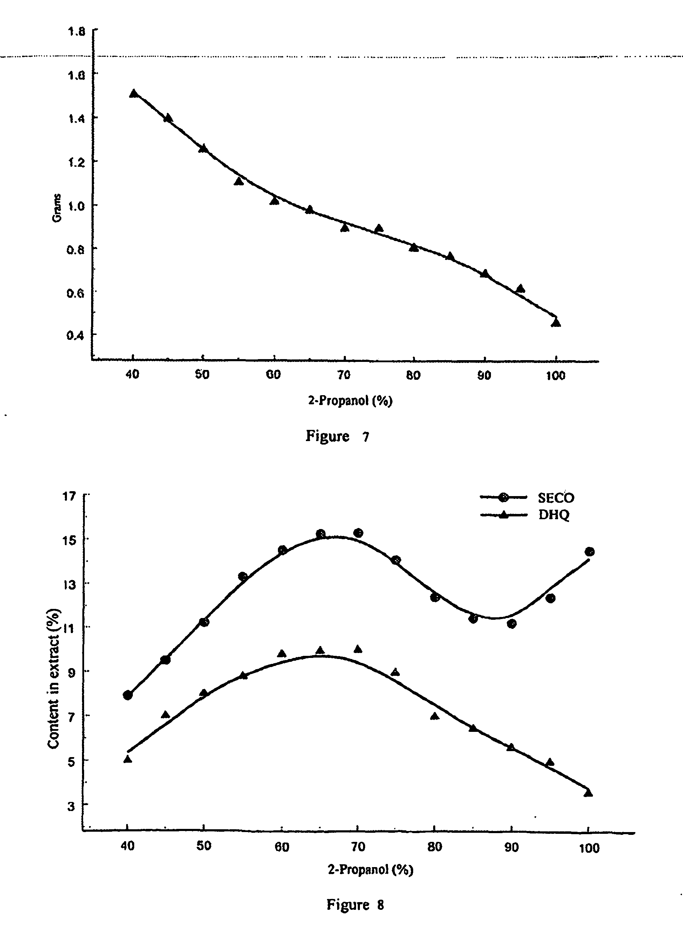

The examples, which do not restrict the terms of the invention, equipped with the accompanying figures, are presented below:
the chromatogram of the extract from sawdust of the larch "white" knots is shown in **Fig. 1****;**
the chromatogram of the extract from sawdust of the "black" knots is presented in **Fig. 2****;**
the results of extraction of the larch knot mass ("white knots") with acetone and water-acetone mixtures are presented in **Fig. 3****,** and the weight amounts of DHQ and SECO in the extracts are shown;
the results of extraction of the larch knot mass ("white knots") with acetone and water-acetone mixtures are presented in **Fig. 4****,** and the total weight of the extracts after solvent evaporation is shown;
the results of extraction of the larch knot mass ("white knots") with acetone and water-acetone mixtures are presented in **Fig. 5****,** and the contents of DHQ and SECO in the evaporated acetone and water-acetone extracts are shown;
the results of extraction of the larch knot mass ("white knots") with 2-propanol and water-(2-propanol) mixtures are shown in **Fig. 6****,** and the weight amounts of DHQ and SECO in the extracts are shown;
the results of extraction of the larch knot mass ("white knots") with 2-propanol and water-(2-propanol) mixtures are shown in **Fig. 7****,** and the total weight of the extracts after solvent evaporation is shown; and
the results of extraction of the larch knot mass ("white knots") with 2-propanol and water-(2-propanol) mixtures are shown in **Fig. 8****,** and the contents of DHQ and SECO in the evaporated isopropanol and water-(2-propanol) extracts are shown.

The extracts enriched in lignans and flavonoids (lignan-flavonoid complexes), as well as lignans and flavonoids after separation, find wide use for the production of biologically active additives and chemical pharmaceutical products of various types. In this case, the corresponding biological activity of the lignan-flavonoid complexes is determined by the content of the lignan and flavonoid components in them, which is demonstrated by the examples presented below, which do not restrict the terms of the present invention. So, the antioxidant properties of the lignan-flavonoid complexes are determined by the total content of the lignan (SECO) and flavonoid (DHQ) components in them. Table 1 demonstrates the antioxidant properties of DHQ, SECO, and their complexes extracted from the larch knot mass according to the said invention (the experiment is described in Example 18). It is seen from the data in Table 1 that the equimolecular amounts of DHQ and SECO exhibit the same antioxidant activity as the lignan-flavonoid complexes containing the same amount of the lignan (SECO) and flavonoid (DHQ) components. This allows one to use the lignan-flavonold complexes along with their separated lignan (SECO) and flavonoid (DHQ) components in the production of antioxidant chemical pharmaceutical products and biologically active additives associated with the treatment and prophylaxis of cardiovascular diseases and infection lesions, correction of lipid exchange, strengthening of the immune status, and others.

The most important sphere of using the lignan-flavonoid complexes formed according to the present invention and the isolated lignan component (SECO) is the use of the produced chemical pharmaceutical products and biologically active additives designed for the prophylaxis and treatment of estrogen-mediated processes. It is known that SECO and DHQ possess low estrogenic activity; however, under the action of the enzymatic system of microflora, SECO undergoes the transformation into enterolactone (ENL) having estrogenic activity. This determines the use of SECO-containing products as biological precursors of ENL and also can be used for the production of chemical pharmaceutical products and biologically active additives designed for the prophylaxis and treatment of estrogen-mediated processes associated with oncologic diseases, development of hyperplasia, and disturbances of women health, including those caused by menopause. As an example that does not restrict the terms of the present invention, the estrogenic activity of SECO, DHQ, and the SECO and DHQ complexes extracted from the larch knot mass according to the present invention (the

experiment is described in Example 19) is shown in Table 2. The same proliferative effect (experiment with the MCF-8 cells) and the ability to inhibit competitively an analogous effect of the natural hormone estradiol are observed when the preparations containing the same amount of SECO are used.

### EXAMPLES

### Example 1

### Chromatographic analysis of the components of the extracts of the "white knot" and "black knot" masses and the chromatograchic analysis of the components of the extracts.

The samples of the crushed mass (in this and further examples, the results of extraction of particles with the thickness to 1 mm passing through a sieve with a channel diameter of 3 mm are presented for the correct comparison of the experimental data) of the "white" and "black" knots of Siberian larch (*Larix sibirica*, hereinafter larch) bearing living and dead cells were extracted with 70% aqueous acetone at room temperature for 24 h (the sample to solvent ratio is 1:10). The aliquots of the solutions (10 µl) were analyzed by HPLC (E.E. Nifant'ev, M.P. Koroteev, G.Z. Kaziev, V.K. Bel'skii, A.I. Stash, A.A. Grachev, V.M. Men'shov, Yu.E. Tsvetkov, N.E. Nifantiev, "To the Problem of Identification of Dihydroquercetin Flavonoid," Zh. Org. Khim. 76: 161-163 (2006)) on the Ultrasphere ODS column (5 µm, 4.6 mm x 25 cm; Beckman) in an acetonitrile (20%)-water system containing 1 mUL trifluoroacetic acid with a flow rate of 0.8 mL/min using a UV detector (254 nm). The contents of DHQ and SECO in the extracts were quantitatively characterized by the calibration curves obtained by the chromatographic analysis of analytically pure samples of DHQ and SECO.

The analysis of the chromatograms of the extracts (chromatograms are shown in **Figs. 1** and **2**) indicates the contents of DHQ and SECO in the "white" knots of 1.2% and 1.7%, respectively, of the total weight, whereas the "black" knots contain only traces of SECO and the DHQ content in only about 0.5% of the total weight. These data show that the mass of "white" knots is preferential raw materials for the isolation of DHQ and SECO.

### Example 2

### Extraction of the larch knot mass with acetone and water-acetone mixtures and the chromatographic analysis of the components of the extracts.

Standard portions (10 g) of the crushed mass of the larch "white" knots were extracted with 100 ml of acetone or water-acetone mixtures of different composition at room temperature for 24 h. The extracts were filtered through a glass filter (No. 3) and evaporated to dryness. A weighed sample of the dry residue (25 mg) was dissolved in 10 mL of acetonitrile and analyzed by HPLC as described above in Example 1 to determine the contents of DHQ and SECO in the starting sample of the knot mass using the chromatographic characteristics of authentic samples of DHQ and SECO.

The experimental results are summarized in **Figs. 3-5****,** showing the weight amounts of DHQ and SECO in the extracts (**Fig. 3**), the total weight of the extracts after the evaporation of the solvent (**Fig. 4**), and the contents of DHQ and SECO in the evaporated acetone and water-acetone extracts (**Fig. 5**). The analysis of the chromatograms indicates that the most efficient extraction of DHQ and SECO is achieved when extracting with mixtures containing acetone in the interval from 50 to 75%, preferably from 60 to 70%. The contents of DHQ and SECO in the extract decrease with an increase in the acetone content in the extracting agent higher than 75% or with a decrease lower than 50%. In addition, for extraction with mixtures containing more than 50% water, the total weight of the extract increases due to the extraction of non-target components from the knot mass, especially carbohydrate derivatives and other compounds highly soluble in aqueous media, which further Impedes the purification of DHQ and SECO.

### Example 3

### Extraction of the larch knot mass with 2-propanol and water-(2-propanol) mixtures and the chromatographic analysis of the components of the extracts.

Standard portions (10 g) of the crushed mass of the larch "white" knots were extracted with 100 ml of 2-propanol or water-(2-propanol) mixtures of different composition at room temperature for 24 h. The extracts were primarily treated and then analyzed by HPLC as described in Example 2.

The experimental results are summarized in **Figs. 6-8****,** showing the weighed amounts of DHQ and SECO in the extracts (**Fig. 6**), the total weight of the extracts after evaporation of the solvent (**Fig. 7**), and the contents of DHQ and SECO in the evaporated isopropanol and water-(2-propanol) extracts (**Fig. 8**). The analysis of the chromatograms and the data on the total weight of the extracts indicates that the most efficient extraction of DHQ and SECO is achieved when extracting with mixtures containing from 50 to 75% 2-propanol, preferably from 60 to 70%. The contents of DHQ and SECO in the extract decrease with an increase in the 2-propanol content in the extracting agent higher than 75% or with a decrease lower than 50%. In addition, for extraction with mixtures containing more than 50% water, the total weight of the extract increases due to the extraction from the knot mass of non-target components, especially carbohydrate derivatives, which further impedes the purification of DHQ and SECO.

### Example 4

### Study of the cavitation disintegration of the knot mass in water followed by extraction with organic solvents.

Crushed larch knots (300 g) were subjected to the cavitation treatment in 10 L of water at 95 °C according to the method described in the Russian Patent No. 2180566 and Russian Patent No. 2233858. The resulting mixture was cooled to room temperature, and the insoluble components were separated by filtration or centrifugation (2 h at 2000 rpm). Attempts to extract the filtrate, being a stable emulsion, with water-immiscible solvents of different polarity (alkanes, aromatic compounds, ethers, chlorinated solvents) failed, because no layers of the organic and aqueous phases formed even upon the addition of solutions of NaCl and aliphatic alcohols to the mixture, which usually facilitates layer separation during extraction of aqueous solutions. Thus, the method of DHQ and SECO isolation from the knot mass, including the cavitation disintegration in water at the first stage, is not efficient In practice.

### Example 5

### Extraction of the larch knot mass with 70% aqueous acetone.

Crushed larch knots (1 kg) were extracted with 70% aqueous acetone (5 L) at room temperature for 24 h. The solution was filtered and evaporated to dryness to give 87 g of a powdered crude mixture of DHQ and SECO. According to the HPLC data (analysis was carried out under the conditions described in Example 1), the product obtained contains 9.8 g of DHQ and 14.3 g of SECO.

### Example 6

### Extraction of the larch knot mass.

Crushed larch knots were extracted with 70% aqueous acetone as described in Example 5. Water (50 mL) was added to a sample of the obtained product (5 g), and the mixture was refluxed for 1 h with stirring. The hot solution was separated from the oily residue and evaporated to dryness to obtain the crude product (1.8 g) enriched in DHQ and SECO, namely, containing 23% DHQ (414 mg) and 32% SECO (576 mg) (according to the HPLC data under the conditions described in Example 1).

### Example 7

### Extraction of the larch knot mass with 70% aqueous isopropanol.

Crushed larch knots (1 kg) were extracted with 70% aqueous 2-propanol (5 L) at room temperature for 24 h. The solution was filtered and evaporated to dryness to obtain 83 g of a powdered crude product containing DHQ (7.4 g) and SECO (12.2 g) (according to the HPLC data under the conditions described in Example 1).

### Example 8

### Extraction of the larch knot mass with 70% aqueous ethanol.

Crushed larch knots (0.1 kg) were extracted with 70% aqueous ethanol (720 mL) at room temperature for 24 h. The solution was filtered and evaporated to dryness to obtain a powdered crude product (7.12 g) containing DHQ (655 mg) and SECO (810 mg) (according to the HPLC data under the conditions described in Example 1).

### Example 9

### Preparation of a mixture of SECO and DHQ in the approximate 1:1 ratio with a purity of 75% and more.

Crushed larch knots were extracted with 70% aqueous acetone as described in Example 5. An (ethyl acetate)-(petroleum ether) mixture (1:2, 10 mL) was added to a sample of the obtained product (5 g), the resulting mixture was stirred at 50 °C, the temperature was brought to ambient temperature, and the supernatant was separated by decantation (or filtration through a paper or glass filter). The obtained residue was chromatographed on a silica gel layer, eluting a mixture of DHQ or SECO with methyl tert-butyl ether (100 ml). The eluate was evaporated to dryness, and the obtained powder (1.45 g) being, according to the HPLC data (under the conditions described in Example 1), a mixture of DHQ and SECO in the 1:1 ratio with a purity of 75% or more. Mixtures of SECO and DHQ of this purity were analogously isolated when the organic extracts of the knot masses were treated with other aqueous organic mixtures according to the present invention, for instance, formed from the products obtained in Examples 2, 7, 8, and 15-17. In this case, the ratio of SECO and DHQ in the isolated product is determined by their contents in the starting raw materials.

### Example 10

### Preparation of the approximate 1:1 SECO and DHQ mixture with a purity of 75% and more.

Crushed larch knots were extracted with 70% aqueous acetone as described in Example 5. A sample of the obtained product (5 g) was stirred for 10-120 min on heating at the temperature not higher than 50 °C with a mixture (10-30 mL) of a hydrocarbon solvent and a more polar solvent taken in such a ratio that the chromatographic mobility values (retention factors, Rf) of SECO and DHQ on standard silica gel plates during thin-layer chromatography would range from 0 to 0.05. After the end of stirring, the temperature of the mixture was brought to ambient temperature, and the supernatant liquid was separated by decantation (or filtration through a paper or glass filter). The obtained residue was chromatographed on a silica gel layer, eluting a mixture of DHQ and SECO with methyl tert-butyl ether (100 mL). The eluate was evaporated to dryhess to obtain a powder (1.45 g), being, according to the HPLC data (under the conditions described in Example 1), a mixture of DHQ and SECO in the approximate 1:1 ratio with a purity of 75% or more. Technologically accessible aliphatic or aromatic compounds, for instance, individual alkanes, petroleum ether, toluene, and others, can be used as a hydrocarbon solvent. Organic compounds characterized by the dielectric constant from 4 to 25 at 25-30 °C can be used as a polar solvent. For instance, a mixture of ethyl acetate and petroleum ether in a ratio of 1:2 can be used.

Mixtures of SECO and DHQ of the indicated purity were isolated similarly by the treatment of the organic extracts of the knot mass with other aqueous organic mixtures according to the said invention, for instance, formed from the products obtained in Examples 2, 7, 8, and 15-17. The SECO and DHQ ratios in the isolated product are determined by their content in the starting raw materials.

### Example 11

### Preparation of the approximate 1:1 SECO and DHQ mixture with a Purity of 75% and more.

Crushed larch knots were extracted with 70% aqueous acetone as described in Example 5. A sample of the obtained product (5 g) was stirred for 10-60 min on heating at the temperature not higher than 50°C with 10-30 ml of a mixture of a hydrocarbon solvent and a more polar solvent with the dielectric constant from 4 to 25 at 25-30 °C. Technologically accessible aliphatic or aromatic compounds, for instance, individual alkanes, petroleum ether, toluene, and others, can be used as a hydrocarbon solvent. The amounts of the taken polar component depend on its dielectric constant. For instance, at the dielectric constants from 20 to 25, 10 vol.% are used; for the dielectric constant from 10 to 15, 30 vol.% are used; and at the dielectric constant from 4 to 10, 40 vol.% are used. For instance, according to the indicated characteristics of the solvents, the 1:2 (ethyl acetate)-(petroleum ether) mixture can be used.

After the end of stirring, the temperature of the mixture was brought to ambient temperature, and the supernatant liquid was separated by decantation (or filtration through a paper or glass filter). The obtained residue was chromatographed on a silica gel layer, eluting a mixture of DHQ and SECO with methyl tert-butyl ether (100 mL). The eluate was evaporated to dryness, and a powder (1.45 g) was obtained, which represented, according to the HPLC data (under the conditions described in Example 1), a mixture of DHQ and SECO in an approximate ratio of 1:1 with the purity equal to or more than 75%.

Mixtures of SECO and DHQ of the indicated purity were isolated similarly by the treatment of the organic extracts of the knot mass with other aqueous organic mixtures according to the present invention, for instance, formed from the products obtained in Examples 2, 7, 8, and 15-17. The SECO and DHQ ratios in the isolated product are determined by their contents in the starting raw materials.

### Example 12

### Preparation of SECO and DHQ samples with a purity of 75% and more.

Crushed larch knots were extracted with 70% aqueous acetone as described in Example 5. Water (50 mL) was added to a sample of the obtained product, and the mixture was refluxed for 1 h with stirring, the hot solution was separated from an oily residue, the temperature of the solution was brought to ambient temperature, and the organic phase was extracted with chloroform (8x30 mL). The organic phase was separated, dried, and evaporated to dryness to obtain crude SECO (670 mg) with a purity of 75% or more (determined by HPLC under the conditions described in Example 1). The aqueous phase remained after the extraction was extracted with ethyl acetate (3x30 ml), and the extracts were joined, dried, and evaporated to dryness to obtain crude DHQ (481 mg) with a purity of 75% or more (determined by HPLC under the conditions described in Example 1). Mixtures of SECO and DHQ of the indicated purity were isolated similarly by the treatment of the organic extracts of the knot mass with other aqueous organic mixtures according to the said invention, for instance, those formed from the products obtained in Examples 2, 7, 8, and 15-17. The SECO and DHQ ratios in the isolated product are determined by their contents in the starting raw materials.

### Example 13

### Preparation of DHQ with a purity of 95-97% and more.

DHQ with a purity of 95-97% and more was obtained by the recrystallization of the raw materials (Sample 12). For instance, a DHQ sample (0.48 g) obtained under the conditions of Example 12 was dissolved in deaerated water (5 mL) at 70-80°C. The resulting solution was cooled to 4°C, and the mixture was stored until the end of crystallization. The precipitated crystals were filtered off and dried *in vacuo* to obtain 0.31 g of DHQ of the indicated purity monitored by HPLC under the conditions described in Example 1. ¹³C NMR spectral data (δ, ppm; DMSO-d₆; Bruker WM-250, 62.9 MHz): 71.6 (C3), 83.1 (C2), 95.0 (C8), 96.0 (C6), 100.5 (C10), 115.2 (C5'), 115.4 (C2'), 119.5 (C1'), 145.0 (C4'), 145.8 (C3'), 162.6 (C9), 163.4 (C5), 166.8 (C7), 197.9 (C4).

### Example 14

### Preparation of SECO with a purity of 95-97% and more.

SECO of 95-97% purity and more was prepared by the recrystallization of the crude product (Example 12). For instance, a SECO sample (660 mg) obtained under the conditions of Example 12 was dissolved in diethyl ether (7 mL) at 30°C, the solution was cooled to 4°C, and the mixture was stored until the end of crystallization. The precipitated crystals were filtered off and dried *in vacuo.* SECO (405 mg) of the indicated purity monitored by the HPLC data under the conditions described in Example 1 was obtained. ¹³C NMR spectral data (δ, ppm; DMSO-d₆; Bruker WM-250, 62.9 MHz): 34.0 (C3,3'), 42.5 (C2,2'), 55.5 (OMe), 60.3 (C1,1'), 113.0 (C6,6'), 115.0 (C9,9'), 121.1 (C5,5'), 132.2 (C4,4'), 144.3 (C7,7'), 147.2 (C8,8').

### Example 15

### Extraction of the knot mass of Siberian fir (Abies sibirica).

Crushed fir knots (100 g) were extracted with 70% aqueous acetone (0.5 L) at room temperature for 24 h. The solution was filtered and evaporated to dryness to obtain 733 mg of a powdered crude mixture of DHQ and SECO. According to the HPLC data (analysis was carried out under the conditions described in Example 1), the product obtained contains 7 mg of DHQ and 91 mg of SECO.

### Example 16

### Extraction of the knot mass of Siberian fir (Abies sibirica).

Crushed fir knots (100 g) were extracted with 70% aqueous 2-propanol (0.5 L) at room temperature for 24 h. The solution was filtered and evaporated to dryness to obtain 655 mg of a powdered crude mixture of DHQ and SECO. According to the HPLC data (analysis was carried out under the conditions described in Example 1), the product obtained contains 6 mg of DHQ and 77 mg of SECO.

### Example 17

### Extraction of the knot mass of Siberian fir (Abies sibirica).

Crushed fir knots (100 g) were extracted with 70% aqueous ethanol (0.5 L) at room temperature for 24 h. The solution was filtered and evaporated to dryness to obtain 725 mg of a powdered crude mixture of DHQ and SECO. According to the HPLC data (analysis was carried out under the conditions described in Example 1), the product obtained contains 6 mg of DHQ and 71 mg of SECO.

### Example 18

### Antioxidant properties of DHQ, SECO, and their complexes.

Antioxidant activity of DHQ, SECO, and their complexes obtained under the conditions of Examples 6 and 7 was studied by a known method (D. J. Jamieson, "Saccharomyces cerevisiae has distinct adaptive responses to both hydrogen peroxide and menadione" J. Bacteriol. 174: 6678-6681 (1992)) on yeast *Saccharomyces cerevisiae* BKM Y-1173. The yeasts were grown to the mid-logarithmic growth stage on Ryder's medium containing 2% glucose, the yeast extract, and mineral salts. The yeast cells were separated from the medium by centrifugation, two times washed with water, and incubated with hydrogen peroxide and antioxidants In an aqueous suspension (OD₆₀₀ 0.1-0.15) for 1 h with periodic shaking. Then the corresponding dilutions were made, the yeasts were seeded on the agarized glucose-peptone medium and cultivated for 2 h at 30°C, and the number of grown colonies and the survival rate of *S. cerevisiae* cells were counted (Table 1).

**Table 1. Influence of the antioxidants on the survival rate of S. cerevisiae cells under the action of hydrogen peroxide**

| Antioxidant | Survival rate, % of test | | | | |
|---|---|---|---|---|---|
| | No antioxidant | DHQ (Example 13, | SECO (Example 14), | DHQ+SECO (Example 6), | DHQ+SECO (Example 7), |
| | | 5 mg/ml | 5 mg/ml | 5 mg/ml** | 5 mg/ml** |
| Test (no H₂O₂) | 100 | 100 | 100 | 100 | 100 |
| 5 mM H₂O₂ | 7±2 | 35±5 | 35±5 | 35±5 | 35±5 |
| 10 mM H₂O₂ | 1* | 9±2 | 9±1 | 9±2 | 9±2 |

| | | | | | |
|---|---|---|---|---|---|
| * The accuracy threshold for counting colonies is 1%. ** The total concentration of DHQ and SECO in the solution is given. | | | | | |

### Example 19

### Comparative study of the proliferation of the human breast cancer cells MCF-7 under the action of estradiol, SECO, and SECO-containing extracts.

The comparative study of the proliferation of the human breast cancer cells MCF-7 under the action of estradiol, SECO, and SECO-containing extracts was carried out according to a known method (H. Adlercreutz, Y. Mousavi, "Enterolactone and estradiol inhibit each other's proliferative effect on MCF-7 breast cancer cells in culture," J. Steroid. Biochem. Mol. Biol. 41: 615-619 (1992)). To estimate the estrogenic activity of the preparations containing SECO, 1 M solutions of the latter in ethanol (the concentration is indicated for SECO present in the solution) were added to the cell culture with achievement of the final 1 µM concentration of SECO. In each experiment on proliferation, 17 β-estradiol was used as a positive standard for the estimation of the estrogenic activity and was also added as an ethanolic solution to achieve the final 1 nM concentration. To estimate the antiestrogenic activity, solutions of 17 β-estradiol and the tested SECO preparations, among which there were the preparations obtained under the conditions of Examples 6, 7, and 14, were simultaneously added to the cell culture. The cell proliferation was estimated by counting the number of cells with the Coulter counter, and the results obtained are summarized in Table 2. In the test experiment (see entry 1 in Table 2), ethanol without lignan was added. Five measurements were carried out under each type of conditions.

**Table 2. Study of the estrogenic and antiestrogenic activity of the preparations containing SECO on the MCF-7 cell culture (see Example 19).**

| Entry | Preparation | Proliferation of cells MCF-7, % |
|---|---|---|
| 1 | Test experiment in the presence of ethanol | 100 ± 10 |
| 2 | Solution of 17 β-estradiol in ethanol | 140 ± 15 |
| 3 | Solution of SECO (Example 6) in ethanol | 115 ± 15 |
| 4 | Solution of SECO (Example 7) in ethanol | 120 ± 15 |
| 5 | Solution of SECO (Example 14) in ethanol | 115 ± 10 |
| 6 | Solution of 17 β-estradiol and SECO (Example 6) in ethanol | 115 ± 15 |
| 7 | Solution of 17 β-estradiol and SECO (Example 7) in ethanol | 120 ± 15 |
| 8 | Solution of 17 β-estradiol and SECO (Example 14) in ethanol | 115 ± 10 |

## Claims

1. The method for the isolation of secoisolariciresinol and dihydroquercetin from wood wherein the crushed wood of the knot zone is extracted with a non-splitting mixture of an organic solvent with water at a solvent content of 50-75% to obtain the extract containing secoisolariciresinol and dihydroquercetin, and the extract is treated to remove the solvent to obtain the final mixture containing secoisolariciresinol and dihydroquercetin.

2. The method according to claim 1 differed by the use of "white knots" as the knot zone of the wood.

3. The method according to claim 1 differed by the use of acetone as an organic solvent.

4. The method according to claims 1 differed by the use of 2-propanol as an organic solvent.

5. The method according to claims 1 differed by the use of ethanol as an organic solvent.

6. The method according to any of claims 1 and 3-5 differed by that the content of the organic solvent in a mixture with water ranges from 60 to 70%.

7. The method for the isolation of secoisolariciresinol and dihydroquercetin from wood wherein the crushed wood of the knot zone is extracted with a non-splitting mixture of an organic solvent with water at a solvent content of 50-75% to obtain the extract containing secoisolariciresinol and dihydroquercetin, and the extract is treated to remove the solvent to obtain the final mixture containing secoisolariciresinol and dihydroquercetin, which is subjected to selective extraction and crystallization to isolate secoisolariciresinol and dihydruquercetin.

8. The method according to claim 7 differed by the use of "white knots" as the knot zone of the wood.

9. The method according to claim 7 differed by the use of acetone as an organic solvent.

10. The method according to claim 7 differed by the use of 2-propanol as an organic solvent.

11. The method according to claim 7 differed by the use of ethanol as an organic solvent.

12. The method according to any of claims 7 and 9-11 differed by that the content of the organic solvent in a mixture with water ranges from 60 to 70%.

13. The method for the isolation of secoisolariciresinol and dihydroquercetin from wood wherein the crushed wood of the knot zone is extracted with a non-splitting mixture of an organic solvent with water at a solvent content of 50-75% to obtain the extract containing secoisolariciresinol and dihydroquercetin, then the extract is treated to remove nonpolar components, the supernatant liquid is removed, and the resulting residue is chromatographed on a silica gel layer followed by the removal of the eluent to obtain a dry mixture of secoisolariciresinol and dihydroquercetin.

14. The method according to claim 13 differed by the use of "white knots" as the knot zone of the wood.

15. The method according to claim 13 differed by the use of acetone as an organic solvent.

16. The method according to claim 13 differed by the use of 2-propanol as an organic solvent.

17. The method according to claim 13 differed by the use of ethanol as an organic solvent.

18. The method according to any of claims 13 and 15-17 differed by that the content of the organic solvent in a mixture with water ranges from 60 to 70%.

19. A mixture of secoisolariciresinol and dihydroquercetin prepared according to any of claims 1, 7 or 13 for use as components of biologically active additives and chemical pharmaceutical products.

## Patentansprüche

1. Verfahren zur Isolation von Secoisolariciresinol und Dihydroquercetin aus Holz, wobei das zerkleinerte Holz des Astknotenbereiches mit einer nicht aufspaltenden Mischung aus einem organischen Lösemittel und Wasser mit einem Lösemittelgehalt von 50 bis 75 % extrahiert wird, um das Extrakt zu erhalten, das Secoisolariciresinol und Dihydroquercetin enthält, und das Extrakt behandelt wird, um das Lösemittel zu entfernen, um die fertige Mischung zu erhalten, die Secoisolariciresinol und Dihydroquercetin enthält.

2. Verfahren nach Anspruch 1, unterschieden durch die Verwendung von verwachsenen Ästen als Astknotenbereich des Holzes.

3. Verfahren nach Anspruch 1, unterschieden durch die Verwendung von Aceton als ein organisches Lösemittel.

4. Verfahren nach Anspruch 1, unterschieden durch die Verwendung von 2-Propanol als ein organisches Lösemittel.

5. Verfahren nach Anspruch 1, unterschieden durch die Verwendung von Ethanol als ein organisches Lösemittel.

6. Verfahren nach einem der Ansprüche 1 und 3 bis 5, unterschieden **dadurch**, dass der Gehalt an organischem Lösemittel in einer Mischung mit Wasser im Bereich von 60 bis 70 % liegt.

7. Verfahren zur Isolation von Secoisolariciresinol und Dihydroquercetin aus Holz, wobei das zerkleinerte Holz des Astknotenbereiches mit einer nicht aufspaltenden Mischung aus einem organischen Lösemittel und Wasser mit einem Lösemittelgehalt von 50 bis 75 % extrahiert wird, um das Extrakt zu erhalten, das Secoisolariciresinol und Dihydroquercetin enthält, und das Extrakt behandelt wird, um das Lösemittel zu entfernen, um die fertige Mischung zu erhalten, die Secoisolariciresinol und Dihydroquercetin enthält, die einer selektiven Extraktion und Kristallisation unterzogen wird, um Secoisolariciresinol und Dihydroquercetin zu isolieren.

8. Verfahren nach Anspruch 7, unterschieden durch die Verwendung von verwachsenen Ästen als Astknotenbereich des Holzes.

9. Verfahren nach Anspruch 7, unterschieden durch die Verwendung von Aceton als ein organisches Lösemittel.

10. Verfahren nach Anspruch 7, unterschieden durch die Verwendung von 2-Propanol als ein organisches Lösemittel.

11. Verfahren nach Anspruch 7, unterschieden durch die Verwendung von Ethanol als ein organisches Lösemittel.

12. Verfahren nach einem der Ansprüche 7 und 9 bis 11, unterschieden **dadurch**, dass der Gehalt an organischem Lösemittel in einer Mischung mit Wasser im Bereich von 60 bis 70 % liegt.

13. Verfahren zur Isolation von Secoisolariciresinol und Dihydroquercetin aus Holz, wobei das zerkleinerte Holz des Astknotenbereiches mit einer nicht aufspaltenden Mischung aus einem organischen Lösemittel und Wasser mit einem Lösemittelgehalt von 50 bis 75 % extrahiert wird, um das Extrakt zu erhalten, das Secoisolariciresinol und Dihydroquercetin enthält, das Extrakt dann behandelt wird, um nicht-polare Komponenten zu entfernen, wobei die überschüssige Flüssigkeit entfernt wird, und der entstandene Rückstand auf einer Silicagelschicht chromatographiert wird, gefolgt vom Entfernen des Eluents, um eine Trockenmischung aus Secoisolariciresinol und Dihydroquercetin zu erhalten.

14. Verfahren nach Anspruch 13, unterschieden durch die Verwendung von verwachsenen Ästen als Astknotenbereich des Holzes.

15. Verfahren nach Anspruch 13, unterschieden durch die Verwendung von Aceton als ein organisches Lösemittel.

16. Verfahren nach Anspruch 13, unterschieden durch die Verwendung von 2-Propanol als ein organisches Lösemittel.

17. Verfahren nach Anspruch 13, unterschieden durch die Verwendung von Ethanol als ein organisches Lösemittel.

18. Verfahren nach einem der Ansprüche 13 und 15 bis 17, unterschieden **dadurch**, dass der Gehalt an organischem Lösemittel in einer Mischung mit Wasser in einem Bereich von 60 bis 70 % liegt.

19. Mischung aus Secoisolariciresinol und Dihydroquercetin, die gemäß einem der Ansprüche 1, 7 oder 13 hergestellt wurde, zur Verwendung als Komponente von biologisch aktiven Additiven und chemisch-pharmazeutischen Produkten.

## Revendications

1. Procédé pour isoler du secoisolaricirésinol et de la dihydroquercétine à partir du bois selon lequel le bois broyé provenant de la zone des branches est extrait au moyen d'un mélange non dissociable d'un solvant organique avec de l'eau à teneur en solvant étant de 50 à 75%, de manière à obtenir un extrait contenant du secoisolaricirésinol et de la dihydroquercétine., et l'extrait est traité de manière à en évacuer le solvant pour obtenir le mélange final contenant le secoisolariciresinol et la dihydroquercétine.

2. Procédé selon les revendications 1, qui se différencie par l'usage de «noeuds blanches» en tant que bois provenant de la zone des branches.

3. Procédé selon la revendication 1 qui se différencie par l'usage d'acétone en tant que solvant organique.

4. Procédé selon la revendication 1 qui se différencie par l'usage de 2-propanol en tant que solvant organique

5. Procédé selon la revendication 1, qui se différencie par l'usage d'éthanol en tant que solvant organique.

6. Procédé selon l'une quelconque des revendications 1 et 3-5 qui se différencie en ce que la teneur en solvant organique dans un mélange avec de l'eau est de 60 à 70%.

7. Procédé pour isoler du secoisolaricirésinol et de la dihydroquercétine à partir du bois selon lequel le bois broyé provenant de la zone des branches est extrait avec un mélange non dissociable d'un solvant organique avec de l'eau à teneur en solvant étant de 50 à 75% de manière à obtenir l'extrait contenant du secoisolaricirésinol et de la dihydroquercétine, et l'extrait est traité de manière à en évacuer le solvant pour obtenir le mélange final contenant du secoisolaricirésinol et de la dihydroquercétine, ledit mélange étant soumis à une extraction sélective et une cristallisation, de manière à isoler le secoisolariciresinol et la dihydroquercétine.

8. Procédé selon les revendication 7 qui se différencie par l'usage de "noeuds blanches" en tant que bois provenant des branches.

9. Procédé selon la revendication 7 qui se différencie par l'usage d'acétone en tant que solvant organique.

10. Procédé selon la revendication 7 qui se différencie par l'usage de 2-propanol en tant que solvant organique.

11. Procédé selon la revendication 7 qui se différencie par l'usage d'éthanol en tant que solvant organique.

12. Procédé selon l'une quelconque des revendications 7 et 9-11, qui se différencie en ce que la teneur en solvant organique dans un mélange avec de l'eau est de 60% à 70%.

13. Procédé pour isoler du secoisolaricirésinol et de la dihydroquercétine à partir du bois selon lequel le bois broyé provenant de la zone des branches est extrait avec un mélange non dissociable d'un solvant organique avec de l'eau à teneur en solvant étant de 50 à 75%, de manière à obtenir un extrait contenant du secoisolaricirésinol et de la dihydroquercétine, on traite l'extrait de manière à en éliminer des composants non polaires, à évacuer le liquide au-dessus du sédiment puis on chromatographie le résidu obtenu sur une couche de gel de silice; on élimine ensuite l'éluat et l'on obtient un mélange sec de secoisolaricirésinol et de dihydroquercétine.

14. Procédé selon les revendication 13 qui se différencie par l'usage de "noeuds blanches" en tant que bois provenant de la zone des branches.

15. Procédé selon la revendication 13 qui se différencie par l'usage d'acétone en tant que solvant organique.

16. Procédé selon la revendication 13 qui se différencie par l'usage de 2-propanol en tant que solvant organique.

17. Procédé selon la revendication 13 qui se différencie par l'usage d'éthanol en tant que solvant organique.

18. Procédé selon l'une quelconque des revendications 13 et 15-17 qui se différencie en ce que la teneur en solvant organique dans un mélange avec de l'eau est de 60% à 70%.

19. Un mélange de secoisolaricirésinol et dihydroquercétine préparé selon l'une quelconque des revendications 1, 7 ou 13 pour l'usage sous forme de composants biologiquement actifs et de produits chimiques et pharmaceutiques.
